# EUROPEAN PATENT APPLICATION

(11) **EP 4 570 179 A1**
(43) Date of publication of application: **18.06.2025**
(21) Application number: 23851579.5
(22) Date of filing: 24.07.2023
(51) Int. Cl.: A61B 6/03, E06B 5/00

(54) **RAY SOURCE MECHANISM AND STATIC COMPUTED TOMOGRAPHY EQUIPMENT**

(30) Priority: 08.08.2022 CN 202210944824
(71) Applicant: Tsinghua University, Haidian District, Beijing 100084 (CN); NUCTECH COMPANY LIMITED, Beijing 100084 (CN)
(72) Inventor: CHEN, Zhiqiang, Beijing 100084 (CN); ZHANG, Li, Beijing 100084 (CN); LI, Yuanjing, Beijing 100084 (CN); HUANG, Qingping, Beijing 100084 (CN); JI, Chao, Beijing 100084 (CN); ZHOU, Yong, Beijing 100084 (CN); DING, Hui, Beijing 100084 (CN)
(74) Representative: Cabinet Beau de Loménie
(86) International application number: PCT/CN2023/108790
(87) International publication number: WO 2024/032357

(57) **Abstract**

A radiation source mechanism is provided, including: a body (22) for outputting rays; a pulling assembly (21) for limiting a position of the body (22) relative to a machine frame (1) and allow the body (22) to rotate about a first axis of the pulling assembly (21) between a closed position close to the machine frame (1) and an open position away from the machine frame (1); and a first support assembly (23) installed at a lower end of the body (22) to support the lower end of the body (22) to smoothly rotate around the first axis relative to a first working surface below the first support assembly (23). A part of the first support assembly (23) has a disengagement state of being disengaged from the first working surface, and a contact state of being in contact with the first working surface in a process of the body (22) disengaging from the closed position, so as to adjust a contact area between the first support assembly (23) and the first working surface. A computer tomography device is further provided, including a machine frame (1), a detector mechanism (3) and a radiation source mechanism (2), the radiation source mechanism (2) is movable between a closed position of shielding the detector mechanism (3) and an open position of exposing the detector mechanism (3).

## Description

### TECHNICAL FIELD

At least one embodiment of the present disclosure relates to a static computer tomography device, and in particular, to a radiation source mechanism and a static computer tomography device.

### BACKGROUND

In a static computer tomography device (static CT), a detector mechanism is typically arranged between a radiation source mechanism and a channel due to an optical path arrangement. When it is required to replace and/or maintain the detector mechanism, it may be difficult to directly contact the detector mechanism from the outside due to an obstruction of the radiation source mechanism. Therefore, it is required to remove the radiation source mechanism, and reset the radiation source mechanism after the replacement and/or maintenance is completed.

At present, the radiation source mechanism used in the static computer tomography device has a large size and a large weight. When moving the radiation source mechanism, it is required to lift the radiation source mechanism by a lifting device (such as an overhead crane, a crane, a gantry crane, etc.), and several people are required to reset the radiation source mechanism to ensure a relative position of the radiation source mechanism and the detector mechanism, so as to keep a resetting accuracy of the radiation source mechanism.

### SUMMARY

In order to solve at least one of the above-mentioned and other technical problems in the related art, the present disclosure provides a radiation source mechanism and a static computer tomography device, in which the radiation source mechanism may be moved by pushing and then a detector mechanism covered by the radiation source mechanism may be installed, repaired or replaced.

In order to achieve the above-mentioned purposes, the embodiments of the present disclosure provides a radiation source mechanism installed on a machine frame of a computer tomography device, comprising: a body configured to output rays; a pulling assembly configured to limit a position of the body relative to the machine frame and allow the body to rotate about a first axis of the pulling assembly between a closed position close to the machine frame and an open position away from the machine frame; and a first support assembly installed at a lower end of the body to support the lower end of the body to smoothly rotate around the first axis relative to a first working surface located below the first support assembly. A part of the first support assembly has a disengagement state of being disengaged from the first working surface, and a contact state of being in contact with the first working surface in a process of the body disengaging from the closed position, so as to adjust a contact area between the first support assembly and the first working surface.

In an exemplary embodiment, when the first support assembly is in the contact state, a rolling friction is formed between the first support assembly and the first working surface.

In an exemplary embodiment, the pulling assembly comprises: a first connecting member installed on the body; and a pivoting portion pivotally arranged between the first connecting member and the machine frame to support the body to rotate between the closed position and the open position.

In an exemplary embodiment, the pulling assembly further comprises a first locking member, and the first locking member is configured to limit a movement of the first connecting member relative to the machine frame when the pivoting portion is in the closed position.

In an exemplary embodiment, the first connecting member is constructed as a block structure, and the first connecting member is arranged on the body for connection with the pulling assembly.

In an exemplary embodiment, the first connecting member is constructed as a frame structure, and the first connecting member comprises: a bottom plate installed on an upper end of the body; a side plate orthogonal to the bottom plate, wherein the side plate is attached to an installation surface of the machine frame through the first locking member; and a longitudinal plate orthogonal to both the bottom plate and the side plate, wherein the longitudinal plate is connected to one end of the pivoting portion.

In an exemplary embodiment, the first locking member comprises a first bolt detachably arranged between the side plate and the machine frame, and the first bolt is configured to keep the first connecting member in a position in contact with the machine frame.

In an exemplary embodiment, the pivoting portion comprises: a first hinge seat arranged on the machine frame; and a pull rod having one end pivotally arranged on the first hinge seat and the other end connected to the first connecting member, wherein the first axis is defined by a shaft of the pull rod rotating around the first hinge seat.

In an exemplary embodiment, the pivoting portion further comprises a second hinge seat installed on an end face of the first connecting member facing the pull rod, and the other end of the pull rod is pivotally installed on the second hinge seat so that the pull rod has a degree of freedom to rotate around a second axis of the second hinge seat.

In an exemplary embodiment, an upper end surface of the first working surface is provided with at least one arc-shaped groove, an arc center of the arc-shaped groove is located on the first axis, and the first support assembly comprises: a plate-shaped member configured to place and support the body, wherein at least part of the plate-shaped member is provided with a through hole at a position corresponding to each of the at least one arc-shaped groove; and a telescopic portion arranged at a position of the plate-shaped member corresponding to at least part of at least one through hole.

In an exemplary embodiment, the telescopic portion comprises a universal ball provided at an end portion of the telescopic portion facing the first working surface, and the universal ball is configured to move between a contact position of being in contact with the first working surface and a disengagement position of being disengaged from the first working surface; and wherein when the first support assembly is in the disengagement state, the universal ball is located in the through hole and in a disengagement position of being disengaged from the arc-shaped groove; and when the first support assembly is in the contact state, at least part of the universal ball extends out of the through hole to abut against a groove bottom of the arc-shaped groove and is in a contact position of rolling along the arc-shaped groove with a rotation of the body.

In an exemplary embodiment, the telescopic portion further comprises: a base arranged on the plate-shaped member; a screw rod sleeved in the base and threadedly mated with the base; and a universal ball seat arranged at a lower end portion of the screw rod, wherein the universal ball is arranged in the universal ball seat.

In an exemplary embodiment, the first support assembly further comprises a second connecting member configured to limit the body on the plate-shaped member.

In an exemplary embodiment, an end portion of the second connecting member facing the body protrudes from an upper end surface of the plate-shaped member and extends into the body to limit the body on the plate-shaped member.

In an exemplary embodiment, the first support assembly further comprises a second locking member, and the second locking member is detachably arranged between the plate-shaped member and the first working surface to limit a relative position of the plate-shaped member and the first working surface.

In an exemplary embodiment, the first working surface is further provided with at least one auxiliary arc-shaped groove, the auxiliary arc-shaped groove has a same arc center as the arc-shaped groove, and a radius of the auxiliary arc-shaped groove is greater than a radius of the arc-shaped groove.

In an exemplary embodiment, at least part of the plate-shaped member is provided with a through hole at a position corresponding to each of the at least one arc-shaped groove; and wherein the telescopic portion is arranged at a position of the plate-shaped member corresponding to at least part of at least one through hole.

In an exemplary embodiment, the mechanism further includes a second support assembly arranged on a lower end surface of the plate-shaped member, wherein when a part of the plate-shaped member rotates to a state of being disengaged from the first working surface, the second support assembly abuts between the plate-shaped member and a second working surface parallel to the first working surface so as to support the part of the plate-shaped member disengaged from the first working surface relative to the second working surface.

In an exemplary embodiment, the second support assembly comprises a wheel-shaped member, and when a part of the plate-shaped member rotates to the state of being disengaged from the first working surface, the wheel-shaped member abuts against the second working surface to support the plate-shaped member and guide the plate-shaped member to rotate along the second working surface.

In an exemplary embodiment, the second support assembly is detachably installed on the lower end surface of the plate-shaped member.

In an exemplary embodiment, the second support assembly is telescopically arranged on the lower end surface of the plate-shaped member and is configured to move between a retraction position of being disengaged from the second working surface and an extension position of abutting against the second working surface.

The embodiments of the present disclosure further provide a computer tomography device, comprising: a machine frame, wherein a detection channel configured to place a detected object is defined in the machine frame; at least one detector mechanism comprising a plurality of detector arrays arranged on a side wall of the machine frame; and at least one radiation source mechanism according to any one of claims, wherein at least one of the plurality of detector arrays is arranged between the radiation source mechanism and the machine frame. The radiation source mechanism is movable between a closed position of shielding the detector mechanism and an open position of exposing the detector mechanism.

In an exemplary embodiment, an upper end of the machine frame extends outward in a horizontal direction to form a first protruding portion, a lower end of the machine frame forms a second protruding portion parallel to the first protruding portion, the detector mechanism is arranged between the first protruding portion and the second protruding portion, and the first working surface is formed on an upper side of the second protruding portion.

In an exemplary embodiment, two detector mechanisms are respectively arranged on outer sides of two opposite side walls of the machine frame, and two radiation source mechanisms are respectively arranged on outer sides of the two detector mechanisms, each detector mechanism is configured to receive the rays emitted from the radiation source mechanism away from the detector mechanism and penetrating the detected object.

In an exemplary embodiment, the detector mechanism is configured to receive scattered rays scattering from the detected object based on the rays emitted from the detector mechanism.

According to the radiation source mechanism and the static computer tomography device in the present disclosure, the pulling assembly is used to establish a connection between the upper portion of the body and the machine frame, and pull the body to prevent the body from tipping over during the rotation of the body between the closed position and the open position. The first support assembly is arranged between the body and the first working surface. When the body is in the closed position, a part of the first support assembly is in the disengagement state of being disengaged from the first working surface, and at least another part of the first support assembly forms a surface contact with the first working surface, so that a friction force is formed between the first support assembly and the first working surface due to a weight of the first support assembly and the body so as to keep the relative position of the body and the first working surface, which helps maintain the stability of the body. When the body disengages from the closed position and moves to the open position, a part of the first support assembly is in the contact state of being in line contact or point contact with the first working surface, and it is only required to overcome a small friction force to change the position of the body relative to the first working surface, then the body may be moved to the open position by an operator without using a lifting device, and the detector mechanism may be exposed at least partially out of the radiation source mechanism for installation, maintenance or replacement.

### BRIEF DESCRIPTION OF THE DRAWINGS

FIG. 1 shows a perspective view of a static computer tomography device according to an exemplary embodiment of the present disclosure;
FIG. 2 shows a perspective view of a radiation source mechanism according to an exemplary embodiment of the present disclosure;
FIG. 3 shows an enlarged view of portion A shown in FIG. 2;
FIG. 4 shows a partial enlarged view of a first support assembly of the radiation source mechanism of the exemplary embodiment shown in FIG. 2;
FIG. 5 shows a partial cross-sectional view of a telescopic portion of the first support assembly of the exemplary embodiment shown in FIG. 4;
FIG. 6 shows a schematic perspective view of the radiation source mechanism of the exemplary embodiment shown in FIG. 2 rotating to an open state;
FIG. 7 shows a principle diagram of a second support assembly of the radiation source mechanism of the exemplary embodiment shown in FIG. 2; and
FIG. 8 shows a perspective view of a static computer tomography device according to an exemplary embodiment of the present disclosure, in which the radiation source mechanism is in an open state.

Reference numerals in the accompanying drawings are explained as follows: 1. machine frame; 11. first protruding portion; 12. second protruding portion; 13. detection channel; 2. radiation source mechanism; 21. pulling assembly; 211. first hinge seat; 212. pull rod; 213. second hinge seat; 214. first connecting member; 215. first bolt; 216. second bolt; 22. body; 23. first support assembly; 231. plate-shaped member; 232. telescopic portion; 2321. first screw; 2322. universal ball; 2323. base; 2324. screw rod; 2325. universal ball seat; 233. arc-shaped groove; 234. third screw; 235. second connecting member; 236. auxiliary arc-shaped groove; 24. second support assembly; 241. universal wheel; and 3. detector mechanism.

### DETAILED DESCRIPTION OF EMBODIMENTS

In order to make objectives, technical solutions and advantages of the present disclosure more clearly understood, the present disclosure will be further described in detail below in combination with specific embodiments and with reference to the accompanying drawings. Terms used here are for the purpose of describing specific embodiments only and are not intended to limit the present disclosure.

The terms "include", "comprise", etc. used here indicate the presence of a feature, step, operation and/or assembly, but do not exclude the presence or addition of one or more other features, steps, operations or assemblies. All terms used here (including technical and scientific terms) have the meanings generally understood by those skilled in the art, unless otherwise defined. It should be noted that the terms used here shall be interpreted to have meanings consistent with the context of the specification, and shall not be interpreted in an idealized or overly rigid manner.

Herein, unless otherwise specified, directional terms such as "upper", "lower", "left", "right", "inside", "outside", etc. are used to indicate orientations or positional relationships shown based on the accompanying drawings, which are intended to facilitate the descriptions of the present disclosure and not to indicate or imply that a mechanism, element or assembly referred to must have a specific orientation or must be constructed or operated in a specific orientation. It should be understood that when an absolute position of a described object changes, relative positional relationships indicated by these terms may also change accordingly. Therefore, these directional terms may not be understood as limitations to the present disclosure.

In a case of using an expression similar to "at least one of A, B and C", it should be explained according to the meaning of the expression generally understood by those skilled in the art (for example, "a system including at least one of A, B and C" should include but not be limited to a system including A alone, a system including B alone, a system including C alone, a system including A and B, a system including A and C, a system including B and C, and/or a system including A, B and C). In a case of using an expression similar to "at least one of A, B or C", it should be explained according to the meaning of the expression generally understood by those skilled in the art (for example, "a system including at least one of A, B or C" should include but not be limited to a system including A alone, a system including B alone, a system including C alone, a system including A and B, a system including A and C, a system including B and C, and/or a system including A, B and C).

According to a general inventive concept of the present disclosure, a radiation source mechanism and a static computer tomography device are provided.

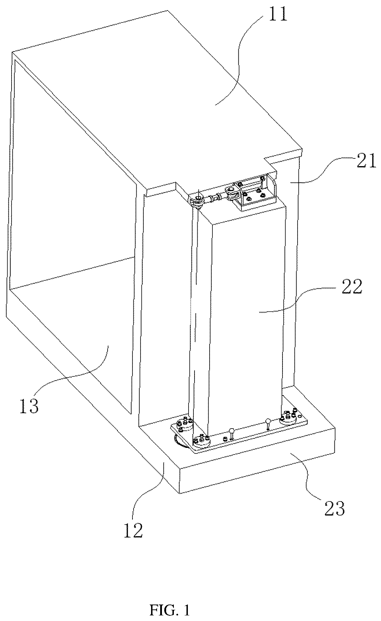

FIG. 1 shows a perspective view of a static computer tomography device according to an exemplary embodiment of the present disclosure.

As shown in FIG. 1, according to an exemplary embodiment of the present disclosure, a static computer tomography device is provided. The static computer tomography device includes a machine frame 1, and a radiation source mechanism is installed on the machine frame 1.

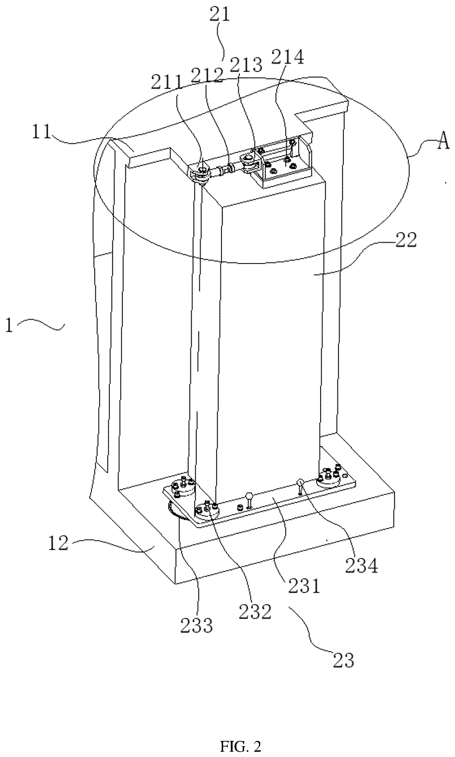

FIG. 2 shows a perspective view of the radiation source mechanism according to an exemplary embodiment of the present disclosure.

As shown in FIG. 2, the exemplary embodiment of the present disclosure provides a radiation source mechanism 2, including a body 22, a pulling assembly 21 and a first support assembly 23. The body 22 is used to output rays. The pulling assembly 21 is used to limit a position of the body 22 relative to the machine frame 1 and allow the body 22 to rotate around a first axis of the pulling assembly 21 between a closed position close to the machine frame 1 and an open position away from the machine frame 1. The first support assembly 23 is installed at a lower end of the body 22 to support the lower end of the body 22 to smoothly rotate around the first axis relative to a first working surface located below the first support assembly 23. A portion of the first support assembly 23 has a disengagement state of being disengaged from the first working surface, and a contact state of being contact with the first working surface in a process of the body 22 disengaging from the closed position, so as to adjust a contact area between the first support assembly 23 and the first working surface.

In an exemplary embodiment, the body 22 may include but not be limited to an X-ray source. It should be noted here that the body 22 is not a key point of protection of the present disclosure, and any radiation source that may be used for a static computer tomography device in the art may be selected and applied, which will not be described in detail here.

In an exemplary embodiment, the pulling assembly 21 is installed between the body 22 and the machine frame 1.

Specifically, the pulling assembly 21 may be connected to at least one of an upper end surface and a side end surface of the body 22, but the present disclosure is not limited thereto.

In an exemplary embodiment, the pulling assembly 21 includes at least one pivot, and an axis of a pivot extending substantially in a vertical direction acts as the first axis.

In an exemplary embodiment, the first support assembly 23 is arranged on a lower end surface of the machine frame 1.

Specifically, a projection of the first support assembly 23 in the vertical direction overlaps at least partially with the lower end surface of the machine frame 1. It should be understood that the embodiments of the present disclosure are not limited thereto.

For example, the first support assembly 23 may be constructed as a frame structure, which is installed around an outer edge of the lower end surface of the body 22 to cover a lower end of the body 22.

In an exemplary embodiment, the first working surface includes but is not limited to any one of an end surface formed on the machine frame, the ground, and an end surface (such as a floor) of a space where the static computer tomography device is placed.

In another exemplary embodiment, the pulling assembly 21 is installed on another frame (not shown) outside the body 22 and the machine frame 1.

Specifically, a frame is provided outside the machine frame 1 at a position facing the body 22, and the pulling assembly 21 is arranged between the frame and the body 22.

Further, the frame may be constructed into but not be limited to the form of a door, an arch, a crossbeam, a longitudinal beam, or any other structural frame suitable for connecting the pulling assembly 21.

In such embodiments, the pulling assembly 21 is used to establish a connection between an upper portion of the body 22 and the machine frame 1, and pull the body 22 to prevent the body from tipping over during a rotation of the body 22 between the closed position and the open position. The first support assembly 23 is arranged between the body 22 and the first working surface. When the body is in the closed position, a portion of the first support assembly is in a disengagement state of being disengaged from the first working surface, and at least another portion of the first support assembly 23 forms a surface contact with the first working surface, so that a friction force is formed between the first support assembly 23 and the first working surface due to a weight of the first support assembly 23 and the body 22, so as to keep the relative position of the body 22 and the first working surface, which helps maintain a stability of the body. When the body 22 is disengaged from the closed position and moves to the open position, a portion of the first support assembly is in a contact state of being in line contact or point contact with the first working surface, and it is only required to overcome a small friction force to change the position of the body 22 relative to the first working surface. In this way, the body may be moved to the open position by an operator without using a lifting device, and a detector mechanism may be exposed at least partially out of the radiation source mechanism for installation, maintenance or replacement.

According to the embodiments of the present disclosure, as shown in FIG. 1 and FIG. 2, the first support assembly 23 is in the contact state, and a rolling friction is formed between the first support assembly 23 and the first working surface.

In such embodiments, when the body is disengaged from the closed position and moves to the open position, a portion of the first support assembly 23 is in a contact state of being contact with the first working surface, and a rolling friction is formed between the first support assembly 23 and the first working surface, so that the body 22 may be moved to the open position by the operator without using the lifting device, and the detector mechanism 3 may be exposed at least partially out of the radiation source mechanism for installation, maintenance or replacement.

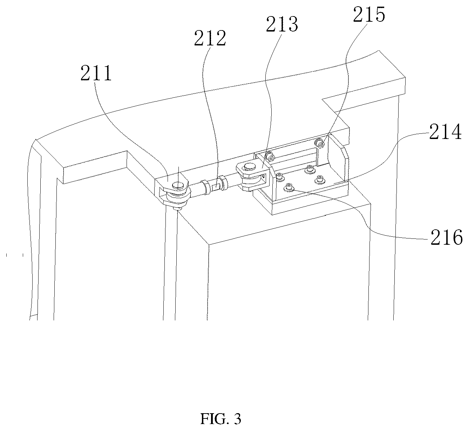

FIG. 3 shows an enlarged view of portion A (including the pulling assembly) shown in FIG. 2.

According to an embodiment of the present disclosure, as shown in FIG. 1 and FIG. 2, the pulling assembly 21 includes a first connecting member 214 and a pivoting portion. The first connecting member 214 is installed on the body 22. The pivoting portion is pivotally arranged between the first connecting member 214 and the machine frame 1 to support the body to rotate between the closed position and the open position.

In an exemplary embodiment, the first connecting member 214 is arranged on an upper end surface of the body 22.

Further, the pivoting portion is arranged between the first connecting member 214 and a side wall of the machine frame 1.

In such embodiments, the first connecting member 214 is installed on the upper end surface of the body 22, so that a side end surface of the body 22 facing the detector mechanism 3 is not blocked. The pivoting portion is used to connect the first connecting member 214 to the machine frame 1 and provide the first connecting member 214 with a degree of freedom to rotate about the first axis.

According to an embodiment of the present disclosure, as shown in FIG. 1 and FIG. 2, the pulling assembly 21 further includes a first locking member, which is used to limit a movement of the first connecting member 214 relative to the machine frame 1 when the pivoting portion is in the closed position.

In an exemplary embodiment, the first locking member is arranged between the first connecting member 214 and the machine frame 1. The first locking member is used to fix the first connecting member 214 to the machine frame 1, and used to keep the first connecting member 214 and the machine frame 1 in a mutually fitting position so as to limit a relative movement of the body 22 and the machine frame 1.

In another exemplary embodiment, the first locking member is arranged on the pivoting portion.

Specifically, the first locking member is arranged on the pivot of the pivoting portion, and is used to keep the body in the closed position by limiting a rotation of the pivot.

Further, the axis of the pivot is defined as the first axis.

In a specific embodiment, the pivot is rotatably provided in a hinge seat, and one axial end of the pivot is axially connected to an output end of a motor. A torque output by the motor may drive the pivot to rotate, and the rotation of the pivot is limited when the motor is turned off.

For another example, a tubular member is sleeved on an outer side of the pivot, and a deep groove bearing is provided between the tubular member and the pivot. The deep groove bearing is filled with magnetorheological grease, and a coil is provided in the tubular member. When the coil is energized, the magnetorheological grease in the bearing forms a Bingham fluid, so that an inner ring and an outer ring of the bearing are in a locked state, and a rotation of the pivot relative to the tubular member is limited.

In such embodiments, the first locking member is used to limit the relative position of the body 22 and the machine frame 1, so that the body 22 is kept in the closed position covering the detector mechanism 3.

According to an embodiment of the present disclosure, the first connecting member 214 is constructed as but not limited to a block structure, which is arranged on the body 22 for connection with the pulling assembly 21.

Specifically, the block structure may include but not be limited to a column structure that is constructed as substantially a cube, a cylinder, an elliptical cylinder and other polygons, which may help an effective and stable connection between the first connecting member 214 and the body 22.

According to an embodiment of the present disclosure, as shown in FIG. 1 to FIG. 3, the first connecting member 214 is constructed as but not limited to a frame structure, and the first connecting member 214 includes a bottom plate, a side plate and a longitudinal plate. The bottom plate is arranged on an upper end of the body 22. The side plate is orthogonal to the bottom plate and is attached to an installation surface of the machine frame 1 through the first locking member. The longitudinal plate is orthogonal to both the bottom plate and the side plate, and is connected to one end of the pivoting portion.

In an exemplary embodiment, the first connecting member 214 includes but is not limited to at least one bottom plate, at least one side plate, and two longitudinal plates.

Specifically, the bottom plate constitutes a bottom surface of a cubic structure, the side plate is provided at one end of the bottom plate facing the machine frame 1, and the longitudinal plates are respectively provided at two ends (left end and right end as shown in FIG. 3) of the side plate.

Further, the bottom plate is fixed to the upper end surface of the body 22 through a second bolt 216.

Furthermore, the bottom plate, the side plate and the longitudinal plate may be connected by any one of connection methods including but not limited to welding, riveting, bolt fixing and integral molding. It should be understood that the embodiments of the present disclosure are not limited thereto.

For example, the first connecting member 214 includes but is not limited to a keel structure and any other structures that may abut against a surface of the machine frame 1 while assembling with the body 22.

In such embodiments, the bottom plate is fixed to the body 22, the side plate is connected to the machine frame 1 through the first locking member, and the longitudinal plate is arranged between the bottom plate and the side plate to enhance a structural strength of the first connecting member 214, which may effectively prevent the body 22 from tipping over in a state of being connected to the machine frame 1.

According to an embodiment of the present disclosure, as shown in FIG. 1 to FIG. 3, the first locking member includes but is not limited to a first bolt 215, which is detachably arranged between the side plate and the machine frame 1 and is used to keep the first connecting member 214 in a position in contact with the machine frame 1.

In an exemplary embodiment, at least part of the machine frame 1 covered by the side plate is provided with a screw hole when the body is in the closed position, and a through hole is formed on the side plate at a position corresponding to an orthographic projection of the screw hole in a horizontal direction, so as to accommodate the first bolt 215 to pass through and be fixed to the machine frame 1.

In such embodiments, when the first bolt 215 is mated with the screw hole, a threaded mating may provide the body with a great tensile resistance, so as to maintain the stability of the body in the closed state.

In another exemplary embodiment, an embedding groove (not shown) is provided on one of the machine frame 1 and the side plate, and an embedding block (not shown) corresponding to the embedding groove in terms of shape and size is provided on the other of the machine frame 1 and the side plate. When the pivoting portion is in a third position, the embedding block is embedded in the embedding groove. A pin hole is provided at one end of the embedding groove, and a pin penetrates the embedding groove through the pin hole and is embedded in the embedding block in a direction orthogonal to an extension direction of the embedding groove, so as to keep the embedding block in the embedding groove.

In such embodiments, a mating of the embedding groove, the embedding block and the pin may be used for a quick connection between the machine frame 1 and the side plate.

According to an embodiment of the present disclosure, as shown in FIG. 1 to FIG. 3, the pivoting portion includes a first hinge seat 211 and a pull rod 212. One end (left end as shown in FIG. 2) of the pull rod 212 is pivotally arranged on the first hinge seat 211, and the other end (right end as shown in FIG. 2) of the pull rod 212 is connected to the first connecting member 214. A shaft of the pull rod 212 rotating around the first hinge seat 211 defines the first axis.

According to an embodiment of the present disclosure, as shown in FIG. 1 to FIG. 3, the pivoting portion further includes a second hinge seat 213, which is installed on an end surface of the first connecting member 214 facing the pull rod. The other end of the pull rod 212 is pivotally installed on the second hinge seat 213, so that the pull rod 212 has a degree of freedom to rotate around a second axis of the second hinge seat 213.

In an exemplary embodiment, the second hinge seat 213 is arranged on the longitudinal plate.

Specifically, two ends of the pull rod 212 are pivotally arranged on the first hinge seat 211 and the second hinge seat 213, respectively.

In an exemplary embodiment, the first hinge seat 211 and the second hinge seat 213 are both configured to rotate around respective hinge shafts in the horizontal direction.

Specifically, an axis of the hinge shaft of the first hinge seat 211 connected to the end portion of the pull rod 212 defines the first axis.

Further, the first axis is located outside an orthographic projection of the body 22 in the vertical direction.

In such embodiments, the first hinge seat 211 and the hinge shaft installed thereon provide the body 22 with a degree of freedom to rotate about the first axis.

In another exemplary embodiment, the first hinge seat 211 and the second hinge seat 213 are both configured to rotate around respective hinge shafts in the horizontal direction.

Specifically, an axis of the hinge shaft of the second hinge seat 213 connected to the end portion of the pull rod 212 defines the second axis.

Further, the second axis is located inside the orthographic projection of the body 22 in the vertical direction.

In such embodiments, the second hinge seat 213 and the hinge shaft installed thereon provide the body 22 with a degree of freedom to rotate about the second axis. On one hand, the second hinge 213 is used to compensate for a difference in mating between the first hinge seat 211, the pull rod 212 and the first connecting member 214 caused by processing accuracy and assembly errors, so that the pull rod 212 may rotate around the first hinge seat 211 at a maximum angle to meet a full movement of the body 22 between the closed position and the open position. On the other hand, if a guide groove is provided between the first support assembly 23 and the first working surface, the body 22 may rotate around the second hinge shaft when it rotates around the first hinge shaft to a particular position, so that the relative position of the body 22 and the machine frame 1 and/or the relative position of the body 22 and the detector mechanism 3 may be adjusted. It should be noted here that a cooperating relationship between the guide groove and the body 22 and/or the first support assembly 23 are/is not key point of protection of the present disclosure, and any cylindrical slider in the art that may mate through a guide groove may be selected and applied, which will not be described in detail here.

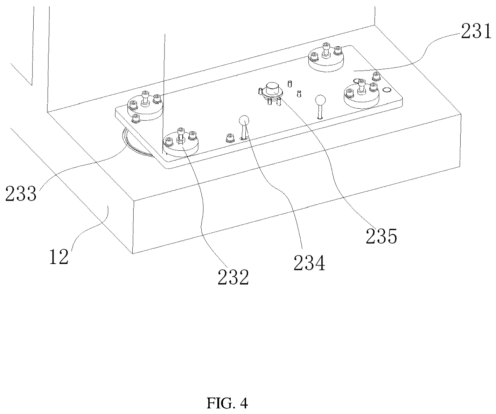

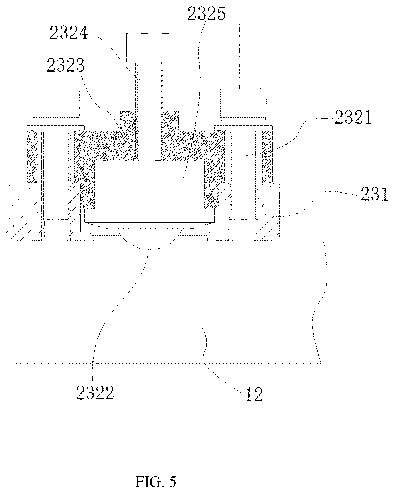

FIG. 4 shows a partial enlarged view of the first support assembly of the radiation source mechanism of the exemplary embodiment shown in FIG. 2. FIG. 5 shows a partial cross-sectional view of a telescopic portion of the first support assembly of the exemplary embodiment shown in FIG. 4.

According to an embodiment of the present disclosure, as shown in FIG. 1, FIG. 2, FIG. 4 and FIG. 5, an upper end surface of the first working surface is provided with at least one arc-shaped groove, an arc center of the arc-shaped groove is located on the first axis, and the first support assembly 23 includes a plate-shaped member 231 and a telescopic portion 232. The plate-shaped member 231 is arranged in the horizontal direction and is used to support the body 22. At least part of the plate-shaped member 231 is provided with a through hole at a position corresponding to each of the at least one arc-shaped groove. The telescopic portion 232 is arranged at a position of the plate-shaped member 231 corresponding to at least part of at least one through hole.

According to an embodiment of the present disclosure, as shown in FIG. 5, the telescopic portion 232 includes a universal ball 2322 provided at an end portion of the telescopic portion 232 facing the first working surface, and the universal ball 2322 is configured to move between a contact position of being in contact with the first working surface and a disengagement position of being disengaged from the first working surface. When the first support assembly 23 is in the disengagement state, the universal ball 2322 is located in the through hole and in a disengagement position of being disengaged from the arc-shaped groove. When the first support assembly 23 is in the contact state, at least part of the universal ball 2322 extends out of the through hole to abut against a groove bottom of the arc-shaped groove and is in a contact position of rolling along the arc-shaped groove with the rotation of the body.

In an exemplary embodiment, the first working surface is provided with two arc-shaped grooves, but the present disclosure is not limited thereto.

Specifically, the two arc-shaped grooves are symmetrically arranged with a projection of the first axis in the vertical direction as a center.

Further, a projection of the through hole provided on the plate-shaped member 231 in the vertical direction is covered by the arc-shaped groove. It should be understood that the embodiments of the present disclosure are not limited thereto.

For example, the first working surface may be provided with one, three, four, five or any other number of arc-shaped grooves.

In an exemplary embodiment, the number of telescopic portions 232 provided on the plate-shaped member 231 is greater than or equal to the number of arc-shaped grooves, so that each arc-shaped groove accommodates at least one universal ball 2322 when the universal ball 2322 of each telescopic portion 232 is in the contact position.

In such embodiments, under the action of the telescopic portion 232, the universal ball 2322 may move between the disengagement position of being disengaged from the arc-shaped groove and the contact position of abutting against the groove bottom of the arc-shaped groove. An extension direction of the arc-shaped groove may define at least part of a translation direction of the radiation source mechanism 2, so as to guide the movement of the radiation source mechanism 2. When the universal ball 2322 is in the disengagement position, a lower end surface of the plate-shaped member 231 is in surface contact with the first working surface. When the body is subjected to a pressure of a horizontal movement, a sliding friction is formed between the plate-shaped member 231 and the first working surface, which may effectively maintain the stability of the radiation source mechanism 2. When the universal ball 2322 is in the contact position, a point contact is formed between the universal ball and the arc-shaped groove, and a rolling friction is formed between the body 22 and the first working surface. In this state, it is only required to overcome a small friction force to translate the radiation source mechanism 2, and the radiation source mechanism 2 may be translated by a manual push (including a pressure applied by the operator through a human body or through a mechanical device) of the operator without using a lifting device.

According to an embodiment of the present disclosure, as shown in FIG. 5, the telescopic portion 232 includes a base 2323, a screw rod 2324 and a universal ball seat 2325. The base 2323 is arranged on the plate-shaped member 231. The screw rod 2324 is sleeved in the base 2323 and threadably mated with the base 2323. The universal ball seat 2325 is arranged at a lower end portion of the screw rod 2324. The universal ball 2322 is arranged in the universal ball seat 2325. It should be understood that the embodiments of the present disclosure are not limited thereto.

For example, the screw rod 2324 may be replaced by mechanisms including but not limited to a cylinder, a lead screw and other mechanisms with a telescopic function.

In an exemplary embodiment, the base 2323 is fixed on the plate-shaped member 231.

Specifically, the base 2323 is fixed on the plate-shaped member 231 by means of a first screw 2321.

Further, the universal ball seat 2325 (including the universal ball 2322) is coaxially fixed to the lower end portion of the screw rod 2324 as shown in FIG. 5. It should be understood that the embodiments of the present disclosure are not limited thereto.

For example, the base 2323 may be fixed on the plate-shaped member 231 by any method including but not limited to bolting, riveting, and welding.

In such embodiments, a threaded mating between the screw rod 2324 and the base 2323 may provide a large torque to push the universal ball 2322 out from a bottom of the through hole so as to abut against the arc-shaped groove.

According to an embodiment of the present disclosure, as shown in FIG. 3, the first support assembly 23 further includes a second connecting member 235, which is used to limit the body 22 on the plate-shaped member 231.

According to an embodiment of the present disclosure, as shown in FIG. 3, an end portion of the second connecting member 235 facing the body 22 protrudes from an upper end surface of the plate-shaped member 231 and extends into the body 22 to limit the body 22 on the plate-shaped member 231.

In an exemplary embodiment, the second connecting member 235 is constructed as but not limited to a cylindrical structure, one end of the cylindrical structure protrudes upward from the lower end surface of the plate-shaped member 231, and the other end of the cylindrical structure extends radially outward to form a flange to abut against an end surface of the plate-shaped member 231 facing the flange.

Further, an embedding groove for accommodating the cylindrical structure is provided at a position of the lower end surface of the body 22 that faces the cylindrical structure extending out of the plate-shaped member 231.

In such embodiments, the second connecting member 235 is embedded in the body 22, which may effectively limit a relative displacement of the body 22 and the plate-shaped member 231 in the horizontal direction, so as to fix the plate-shaped member 231 and the body 22 as a whole.

In another exemplary embodiment, the second connecting member 235 further includes a plurality of second screws, which extend upward from the lower end surface of the plate-shaped member 231 and are arranged around the cylindrical structure to fix the second connecting member 235 to the plate-shaped member 231 and the body 22. It should be understood that the embodiments of the present disclosure are not limited thereto. For example, the body 22 may be fixed on the plate-shaped member 231 by welding, riveting, mortising, or any other method.

According to an embodiment of the present disclosure, as shown in FIG. 4, the first support assembly 23 further includes a second locking member, which is detachably arranged between the plate-shaped member 231 and the first working surface to limit a relative position of the plate-shaped member 231 and the first working surface.

In an exemplary embodiment, the second locking member includes but is not limited to a third screw 234.

Specifically, the plate-shaped member 231 is provided with a through hole, and the first working surface is provided with a positioning hole at a position corresponding to the through hole. When the through hole and the positioning hole are aligned, the third screw 234 may be inserted into the positioning hole to limit the relative position of the plate-shaped member 231 and the first working surface.

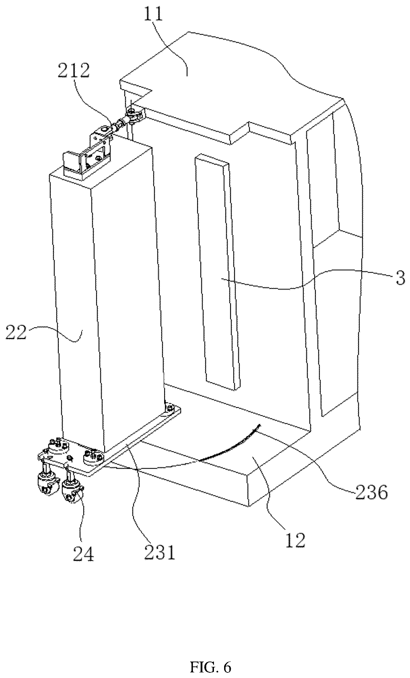

FIG. 6 shows a schematic perspective view of the radiation source mechanism of the exemplary embodiment shown in FIG. 2 rotating to the open state.

According to an embodiment of the present disclosure, as shown in FIG. 6, the first working surface is further provided with at least one auxiliary arc-shaped groove 236, the auxiliary arc-shaped groove 236 has a same arc center as the arc-shaped groove 233, and a radius of the auxiliary arc-shaped groove 236 is greater than a radius of the arc-shaped groove 233.

According to an embodiment of the present disclosure, as shown in FIG. 5 and FIG. 6, at least part of the plate-shaped member 231 is provided with a through hole at a position corresponding to each of the at least one auxiliary arc-shaped groove. The telescopic portion 232 is arranged at a position of the plate-shaped member 231 corresponding to at least part of at least one through hole.

In an exemplary embodiment, the first working surface is provided with two arc-shaped grooves 233, but the present disclosure is not limited thereto.

Specifically, the two arc-shaped grooves 233 are symmetrically arranged to form a substantially ring structure, with a projection of the first axis in the vertical direction as a center.

Further, the first working surface is provided with one auxiliary arc-shaped groove 236, but the present disclosure is not limited thereto.

In an exemplary embodiment, four corners of the plate-shaped member 231 are respectively provided with four telescopic portions 232, but the present disclosure is not limited thereto. The universal balls 2322 of two telescopic portions 232 may abut against the groove bottom and/or a groove wall of the arc-shaped groove 233 when located in the contact positions, and the universal balls 2322 of the other two telescopic portions 232 may abut against a groove bottom and/or a groove wall of the auxiliary arc-shaped groove 236 when located in the contact positions.

In such embodiments, the arc-shaped groove 233, the auxiliary arc-shaped groove 236 and the plurality of telescopic portions 232 are used to disperse a pressure applied by the body 22 to the plate-shaped member 231, so that the radiation source mechanism 2 may subject to a uniform force during a translation process, which helps prevent the body 22 from tipping over due to uneven force, and also helps improve a smoothness of the translation process of the body 22.

According to an embodiment of the present disclosure, as shown in FIG. 6, the radiation source mechanism 2 further includes a second support assembly 24, which is arranged on the lower end surface of the plate-shaped member 231. When a part of the plate-shaped member 231 rotates to a state of being disengaged from the first working surface, the second support assembly 24 abuts between the plate-shaped member 231 and a second working surface parallel to the first working surface so as to support, relative to the second working surface, the part of the plate-shaped member 231 disengaged from the first working surface.

In an exemplary embodiment, the first working surface is characterized as a plane formed by the upper end surface of a second protruding portion 12 of the machine frame.

Further, the second working surface is characterized as a plane formed by the ground below the machine frame.

In such embodiments, when the plate-shaped member 231 rotates to the position shown in FIG. 5, a part (the right end shown in FIG. 6) of the plate-shaped member 231 is disengaged and suspended from the first working surface. The second support assembly 24 extends longitudinally, and two ends of the second support assembly 24 respectively abut against the lower end surface of the plate-shaped member 231 and the second working surface to support the plate-shaped member 231 and the body 22, which may effectively prevent a tip over caused by a center deviation of the body 22 due to a suspension of the plate-shaped member 231.

According to an embodiment of the present disclosure, as shown in FIG. 6, the second support assembly includes but is not limited to a wheel-shaped member. When a part of the plate-shaped member 231 rotates to a state of being disengaged from the first working surface, the wheel-shaped member may abut against the second working surface to support the plate-shaped member 231 and guide the plate-shaped member 231 to rotate along the second working surface.

In an exemplary embodiment, the wheel-shaped member includes but is not limited to a universal wheel 241.

Specifically, the universal wheel 241 is arranged on the lower end surface of the plate-shaped member 231, so as to support the plate-shaped member 231 and the body 22 when one end (the right end as shown in FIG. 6) of the plate-shaped member 231 rotates to a state of being disengaged from the first working surface.

Such embodiments may be applied to usage scenarios where a size of the machine frame 1 is limited. When the machine frame 1 fails to meet the requirement of rotating the plate-shaped member 231 to a complete stroke, the universal wheel 241 may support and guide the plate-shaped member 231 after the plate-shaped member 231 is disengaged from the first working surface, which may help improve the stability of the radiation source mechanism 2 and expand the usage scenarios of the radiation source mechanism 2. It should be understood that the embodiments of the present disclosure are not limited thereto.

For example, the second support assembly may include a cushion block, a support seat, and other structures that may fill a gap formed between the first working surface and the second working surface in the vertical direction.

According to an embodiment of the present disclosure, as shown in FIG. 6, the second support assembly 24 is detachably arranged on the lower end surface of the plate-shaped member 231.

In an exemplary embodiment, the plate-shaped member 231 is preset with a plurality of installation holes. When a position of the installation hole on the plate-shaped member 231 is moved to be disengaged from the first working surface, the operator may place the second support assembly 24 between the lower end surface of the plate-shaped member 231 and the second working surface, and fix the second support assembly 24 in the installation hole.

In such embodiments, the second support assembly 24 has high flexibility, and the second support assembly 24 having a suitable size and strength may be selected by the operator according to actual usage scenarios. Moreover, the second support assembly 24 has a wide adaptability. For example, in a scenario where the second working surface is flat, the universal wheel 241 may be selected as the second support assembly 24. For another example, in a scenario where the second working surface has a partially protruding uneven surface, at least one of a support rod, a cushion block, etc. may be selected as the second support assembly 24 to support the plate-shaped member 231 and the body 22.

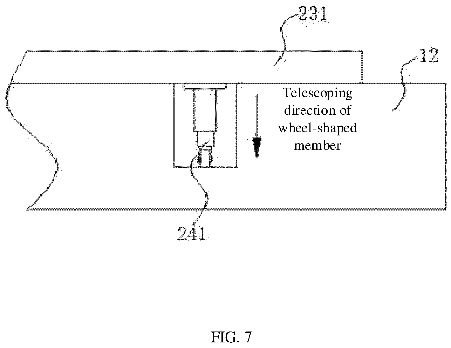

FIG. 7 shows a principle diagram of the second support assembly of the radiation source mechanism of the exemplary embodiment shown in FIG. 2.

According to an embodiment of the present disclosure, as shown in FIG. 7, the second support assembly 24 is telescopically arranged on the lower end surface of the plate-shaped member 231, and is configured to move between a retraction position of being disengaged from the second working surface and an extension position of abutting against the second working surface.

In an exemplary embodiment, the second support assembly 24 includes but is not limited to a telescopic cylinder, an electric push rod, a lead screw, and any other structures having telescoping and jacking functions.

Specifically, a groove is provided on the plate-shaped member 231 and/or the machine frame 1. The second support assembly 24 is located in the groove in the retraction state, and extends from the groove and serves for supporting between the plate-shaped member 231 and the second working surface in the extension state.

In another exemplary embodiment, the second support assembly 24 includes but is not limited to a telescopic universal wheel 241.

Specifically, the universal wheel 241 may be an universal wheel that is telescopic in the vertical direction, an universal wheel that is foldable in the horizontal direction, or an universal wheel or an universal wheel frame mechanism that may have other telescoping functions, but the present disclosure is not limited thereto. It should be noted here that the specific telescoping method of the universal wheel 241 is not a key point of protection of the present disclosure, and any telescopic universal wheel 241 known in the art may be selected and applied, which will not be described in detail here.

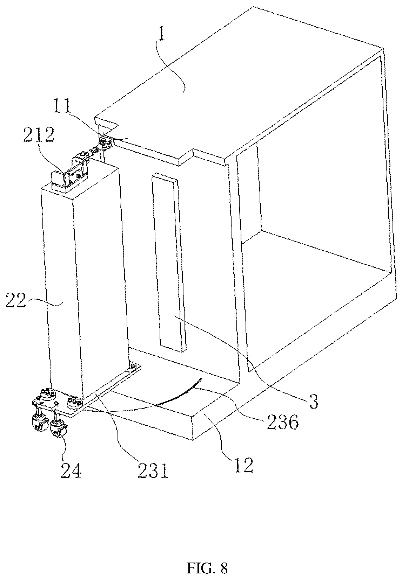

FIG. 8 shows a perspective view of the static computer tomography device according to an exemplary embodiment of the present disclosure, in which the radiation source mechanism is in the open state.

The exemplary embodiments of the present disclosure further provide a static computer tomography device, as shown in FIG. 1 and FIG. 8, including a machine frame 1, at least one detector mechanism 3 and at least one radiation source mechanism 2. A detection channel 13 used to place a detected object is defined in the machine frame 1. The detector mechanism 3 is arranged on a side wall of the machine frame 1, and each detector mechanism 3 includes a plurality of detector arrays. The detector arrays 3 are respectively arranged between the radiation source mechanism 2 and the machine frame 1. The radiation source mechanism 2 is movable between a closed position of shielding the detector mechanism 3 and an open position of exposing the detector mechanism 3.

In such embodiments, the radiation source mechanism 2 is pivotally arranged on the machine frame 1. In the open position, the radiation source mechanism 2 exposes the detector mechanism 3, so that the detector mechanism 3 may be maintained and/or repaired by the operator. In the closed position, the radiation source mechanism 2 is reset to the position of shielding the detector mechanism 3, so that the static computer tomography device may perform tomography work.

According to an embodiment of the present disclosure, as shown in FIG. 8, an upper end of the machine frame 1 extends outward in the horizontal direction to form a first protruding portion 11, and a lower end of the machine frame 1 forms the second protruding portion 12 parallel to the first protruding portion 11. The detector mechanism 3 is arranged between the first protruding portion 11 and the second protruding portion 12, and the first working surface is formed on an upper side of the second protruding portion 12.

In such embodiments, the radiation source mechanism 2 is arranged on the machine frame 1 so that the radiation source mechanism 2 and the detector mechanism 3 have a high degree of integrity, which facilitates an overall installation, maintenance and transportation of the static computer tomography device.

According to an embodiment of the present disclosure, as shown in FIG. 8, the static computer tomography device includes two detector mechanisms 3 respectively arranged on outer sides of two opposite side walls of the machine frame 1, and two radiation source mechanisms 2 are respectively arranged on outer sides of the two detector mechanisms 3. Each detector mechanism 3 is used to receive the rays emitted from the radiation source mechanism 2 away from the detector mechanism 3 and penetrating the detected object.

In such embodiments, the detector mechanism 3 is used to receive the rays output by an opposite radiation source mechanism 2 and penetrating the detected object.

According to an embodiment of the present disclosure, as shown in FIG. 8, the detector mechanism 3 is used to receive scattered rays scattering from the detected object based on the rays emitted from the detector mechanism 3.

In such embodiments, the pulling assembly 21 is arranged on the first protruding portion 11, and the first support assembly 23 is arranged between the second protruding portion 12 and the body 22. When the body is in the closed position, the first support assembly 23 is in the contact position, and a sliding friction extending along the first working surface is formed between the first support assembly 23 and the first working surface due to a weight of the first support assembly 23 and the body, which helps maintain the stability of the body in the closed position. When the body is disengaged from the closed position and moves to the open position, a part of the first support assembly 23 is in the contact state of being in contact with the first working surface, and a rolling friction is formed between the first support assembly 23 and the first working surface. In this way, the body 22 may be moved to the open position by the operator without using lifting device, and the detector mechanism 3 is exposed at least partially out of the radiation source mechanism for installation, maintenance or replacement.

Those skilled in the art may understand that features described in various embodiments of the present disclosure and/or the claims may be combined in various ways, even if such combinations are not explicitly described in the present disclosure. In particular, without departing from the spirit and teachings of the present disclosure, features described in the various embodiments of the present disclosure and/or the claims may be combined in various ways. All these combinations fall within the scope of the present disclosure.

The above specific embodiments further illustrate the objectives, technical solutions and beneficial effects of the present disclosure in detail. It should be understood that the above descriptions are merely specific embodiments of the present disclosure and are not intended to limit the present disclosure. Any modifications, equivalent substitutions, improvements, etc. made within the spirit and principles of the present disclosure should be included in the protection scope of the present disclosure.

## Claims

1. A radiation source mechanism installed on a machine frame (1) of a computer tomography device, comprising:
a body (22) configured to output rays;
a pulling assembly (21) configured to limit a position of the body (22) relative to the machine frame (1) and allow the body (22) to rotate about a first axis of the pulling assembly (21) between a closed position close to the machine frame (1) and an open position away from the machine frame (1); and
a first support assembly (23) installed at a lower end of the body (22) to support the lower end of the body (22) to smoothly rotate around the first axis relative to a first working surface located below the first support assembly (23),
wherein a part of the first support assembly (23) has a disengagement state of being disengaged from the first working surface, and a contact state of being in contact with the first working surface in a process of the body (22) disengaging from the closed position, so as to adjust a contact area between the first support assembly (23) and the first working surface.

2. The radiation source mechanism according to claim 1, wherein when the first support assembly (23) is in the contact state, a rolling friction is formed between the first support assembly (23) and the first working surface.

3. The radiation source mechanism according to claim 1 or 2, wherein the pulling assembly (21) comprises:
a first connecting member (214) installed on the body (22); and
a pivoting portion pivotally arranged between the first connecting member (214) and the machine frame (1) to support the body to rotate between the closed position and the open position.

4. The radiation source mechanism according to claim 3, wherein the pulling assembly (21) further comprises a first locking member, and the first locking member is configured to limit a movement of the first connecting member (214) relative to the machine frame (1) when the pivoting portion is in the closed position.

5. The radiation source mechanism according to claim 4, wherein the first connecting member (214) is constructed as a block structure, and the first connecting member is arranged on the body (22) for connection with the pulling assembly (21).

6. The radiation source mechanism according to claim 4, wherein the first connecting member (214) is constructed as a frame structure, and the first connecting member (214) comprises:
a bottom plate installed on an upper end of the body (22);
a side plate orthogonal to the bottom plate, wherein the side plate is attached to an installation surface of the machine frame (1) through the first locking member; and
a longitudinal plate orthogonal to both the bottom plate and the side plate, wherein the longitudinal plate is connected to one end of the pivoting portion.

7. The radiation source mechanism according to claim 6, wherein the first locking member comprises a first bolt detachably arranged between the side plate and the machine frame (1), and the first bolt is configured to keep the first connecting member (214) in a position in contact with the machine frame (1).

8. The radiation source mechanism according to claim 3, wherein the pivoting portion comprises:
a first hinge seat (211) arranged on the machine frame (1); and
a pull rod (212) having one end pivotally arranged on the first hinge seat and the other end connected to the first connecting member (214),
wherein the first axis is defined by a shaft of the pull rod (212) rotating around the first hinge seat (211).

9. The radiation source mechanism according to claim 8, wherein the pivoting portion further comprises a second hinge seat (213) installed on an end face of the first connecting member (214) facing the pull rod, and the other end of the pull rod (212) is pivotally installed on the second hinge seat (213) so that the pull rod (212) has a degree of freedom to rotate around a second axis of the second hinge seat (213).

10. The radiation source mechanism according to any one of claims 1 to 6, wherein an upper end surface of the first working surface is provided with at least one arc-shaped groove, an arc center of the arc-shaped groove is located on the first axis, and the first support assembly (23) comprises:
a plate-shaped member (231) configured to place and support the body (22), wherein at least part of the plate-shaped member (231) is provided with a through hole at a position corresponding to each of the at least one arc-shaped groove; and
a telescopic portion (232) arranged at a position of the plate-shaped member (231) corresponding to at least part of at least one through hole.

11. The radiation source mechanism according to claim 10, wherein the telescopic portion (232) comprises a universal ball (2322) provided at an end portion of the telescopic portion (232) facing the first working surface, and the universal ball (2322) is configured to move between a contact position of being in contact with the first working surface and a disengagement position of being disengaged from the first working surface; and
wherein when the first support assembly (23) is in the disengagement state, the universal ball (2322) is located in the through hole and in a disengagement position of being disengaged from the arc-shaped groove; and when the first support assembly (23) is in the contact state, at least part of the universal ball (2322) extends out of the through hole to abut against a groove bottom of the arc-shaped groove and is in a contact position of rolling along the arc-shaped groove with a rotation of the body.

12. The radiation source mechanism according to claim 11, wherein the telescopic portion (232) further comprises:
a base (2323) arranged on the plate-shaped member (231);
a screw rod (2324) sleeved in the base (2323) and threadedly mated with the base (2323); and
a universal ball seat (2325) arranged at a lower end portion of the screw rod (2324),
wherein the universal ball (2322) is arranged in the universal ball seat (2325).

13. The radiation source mechanism according to claim 10, wherein the first support assembly (23) further comprises a second connecting member (235) configured to limit the body (22) on the plate-shaped member (231).

14. The radiation source mechanism according to claim 13, wherein an end portion of the second connecting member (235) facing the body (22) protrudes from an upper end surface of the plate-shaped member (231) and extends into the body (22) to limit the body (22) on the plate-shaped member (231).

15. The radiation source mechanism according to claim 10, wherein the first support assembly (23) further comprises a second locking member, and the second locking member is detachably arranged between the plate-shaped member (231) and the first working surface to limit a relative position of the plate-shaped member (231) and the first working surface.

16. The radiation source mechanism according to any one of claims 10 to 15, wherein the first working surface is further provided with at least one auxiliary arc-shaped groove (236), the auxiliary arc-shaped groove (236) has a same arc center as the arc-shaped groove (233), and a radius of the auxiliary arc-shaped groove (236) is greater than a radius of the arc-shaped groove (233).

17. The radiation source mechanism according to claim 16, wherein at least part of the plate-shaped member (231) is provided with a through hole at a position corresponding to each of the at least one arc-shaped groove; and
wherein the telescopic portion (232) is arranged at a position of the plate-shaped member (231) corresponding to at least part of at least one through hole.

18. The radiation source mechanism according to claim 10, further comprising a second support assembly (24) arranged on a lower end surface of the plate-shaped member (231), wherein when a part of the plate-shaped member (231) rotates to a state of being disengaged from the first working surface, the second support assembly (24) abuts between the plate-shaped member (231) and a second working surface parallel to the first working surface so as to support the part of the plate-shaped member (231) disengaged from the first working surface relative to the second working surface.

19. The radiation source mechanism according to claim 18, wherein the second support assembly (24) comprises a wheel-shaped member, and when a part of the plate-shaped member (231) rotates to the state of being disengaged from the first working surface, the wheel-shaped member abuts against the second working surface to support the plate-shaped member (231) and guide the plate-shaped member (231) to rotate along the second working surface.

20. The radiation source mechanism according to claim 18 or 19, wherein the second support assembly (24) is detachably installed on the lower end surface of the plate-shaped member (231).

21. The radiation source mechanism according to claim 18 or 19, wherein the second support assembly (24) is telescopically arranged on the lower end surface of the plate-shaped member (231) and is configured to move between a retraction position of being disengaged from the second working surface and an extension position of abutting against the second working surface.

22. A computer tomography device, comprising:
a machine frame (1), wherein a detection channel (13) configured to place a detected object is defined in the machine frame;
at least one detector mechanism (3) comprising a plurality of detector arrays arranged on a side wall of the machine frame (1); and
at least one radiation source mechanism (2) according to any one of claims 1 to 21, wherein at least one of the plurality of detector arrays is arranged between the radiation source mechanism and the machine frame,
wherein the radiation source mechanism (2) is movable between a closed position of shielding the detector mechanism (3) and an open position of exposing the detector mechanism (3).

23. The computer tomography device according to claim 22, wherein an upper end of the machine frame (1) extends outward in a horizontal direction to form a first protruding portion (11), a lower end of the machine frame (1) forms a second protruding portion (12) parallel to the first protruding portion (11), the detector mechanism (3) is arranged between the first protruding portion (11) and the second protruding portion (12), and the first working surface is formed on an upper side of the second protruding portion (12).

24. The computer tomography device according to claim 22 or 23, wherein two detector mechanisms (3) are respectively arranged on outer sides of two opposite side walls of the machine frame (1), and two radiation source mechanisms (2) are respectively arranged on outer sides of the two detector mechanisms (3), each detector mechanism (3) is configured to receive the rays emitted from the radiation source mechanism (2) away from the detector mechanism (3) and penetrating the detected object.

25. The computer tomography device according to claim 22 or 23, wherein the detector mechanism (3) is configured to receive scattered rays scattering from the detected object based on the rays emitted from the detector mechanism (3).
